# NEUE EUROPÄISCHE PATENTSCHRIFT

(11) **EP 0 674 505 B2**
(45) Veröffentlichungstag und Bekanntmachung des Hinweises auf die Entscheidung über den Einspruch: **12.08.2009**
(45) Hinweis auf die Patenterteilung: 05.08.1998
(21) Anmeldenummer: 94900762.9
(22) Anmeldetag: 07.12.1993
(51) Int. Cl.: A61K 31/415, A61K 8/35, A61K 8/365, A61K 8/49, A61K 8/67, A61Q 19/00, A61Q 19/08

(54) **SYNERGISTISCHE WIRKSTOFFKOMBINATIONEN ZUR KOSMETISCHEN ODER DERMATOLOGISCHEN PFLEGE DER HAUT ODER DER HAUTANHANGSGEBILDE**
SYNERGISTIC COMBINATIONS OF ACTIVE SUBSTANCE FOR THE COSMETIC OR DERMATOLOGICAL CARE OF THE SKIN, HAIR AND NAILS
COMBINAISONS SYNERGIQUES DE PRINCIPES ACTIFS POUR LES SOINS COSMETIQUES OU DERMATOLOGIQUES DE LA PEAU OU DES ELEMENTS TEGUMENTAIRES

(30) Priorität: 18.12.1992 DE 4242876
(43) Veröffentlichungstag der Anmeldung: 04.10.1995
(73) Patentinhaber: Beiersdorf Aktiengesellschaft, 20245 Hamburg (DE)
(72) Erfinder: SAUERMANN, Gerhard, D-24649 Wiemersdorf (DE); SCHÖNROCK, Uwe, D-22851 Norderstedt (DE); SCHREINER, Volker, D-20259 Hamburg (DE); STÄB, Franz, D-21379 Echem (DE)
(86) Internationale Anmeldenummer: PCT/DE1993/001166
(87) Internationale Veröffentlichungsnummer: WO 1994/014412

(56) Entgegenhaltungen:
- EP-A- 0 081 771
- EP-A- 0 330 583
- EP-A- 0 383 467
- EP-A- 0 508 324
- EP-A1- 0 552 516
- WO-A1-92/18116
- DE-A- 3 029 263
- GB-A- 2 120 939
- JP-A2- 03 167 111
- JP-A2- 03 167 112
- JP-A2- 03 167 113
- US- - 4 380 549
- US- - 4 584 191
- US- - 4 737 360
- US- - 5 162 344
- äRZTLICHE KOSMETOLOGIE Bd. 16, Nr. 1 , 1986 , DE Seiten 47 - 54 FAULHABER ET AL. 'Der Einfluss von Sebamed flüssig Waschemulsion aif die ekzematöse Haut'
- ÄRZTLICHE KOSMETOLOGIE Bd. 16, Nr. 1 , 1986 , DE Seiten 47 - 54 FAULHABER ET AL. 'Der Einfluss von Sebamed flüssig Waschemulsion auf die ekzematöse Haut'

## Beschreibung

Die vorliegende Erfindung betrifft kosmetische und/oder dermatologische Wirkstoffkombinationen zur kosmetischen und/oder dermatologischen Pflege der Haut und/oder der Hautanhangsgebilde sowie kosmetische und/oder dermatologische Zubereitungen, solche Wirkstoffkombinationen enthaltend. Ferner betrifft die Erfindung Verfahren zur Pflege und Regeneration der Haut und/oder der Hautanhangsgebilde sowie die Verwendung kosmetischer und/oder dermatologischer Wirkstoffkombinationen zur kosmetischen und/oder dermatologischen Pflege der Haut und/oder der Hautanhangsgebilde.

Zu den Hautanhangsgebilden zählen die Haupt- und Körperhaare sowie insbesondere die Finger- und Fußnägel.

Unter Hautpflege im Sinne der Erfindung ist in erster Linie zu verstehen, daß die natürliche Funktion der Haut als Barriere gegen Umwelteinflüsse (z.B. Schmutz, Fremdstoffe, Mikroorganismen) und gegen den Verlust von körpereigenen Stoffen (z.B. Wasser, Elektrolyte, natürliche feuchtigkeitsbindende Stoffe) gestärkt, unterstützt und, falls nötig, wiederhergestellt wird.

Wird die natürliche Funktion der Haut gestört, kann es zu verstärkter Resorption toxischer und/oder allergener Fremdstoffe oder zum Befall mit pathogenen Mikroorganismen und als Folge zu entzündlichen oder allergischen Hautreaktionen kommen.

Die menschliche Haut verliert durch körpereigene Mechanismen, zum Beispiel durch die Transpiration, ständig eine gewisse Menge an Feuchtigkeit. Aber auch durch äußere Einflüsse wie die tägliche Körperwäsch. Wind und Wetter verliert die Haut wichtige funktionale Bestandteile. Obwohl die gesunde Haut durchaus imstande ist, diesen Verlust auszugleichen, ist es Ziel der Hautpflege, die Haut beim Ausgleich dieses Verlustes zu unterstützen.

Wenn aber das natürliche Regenerationsvermögen der Haut, zum Beispiel infolge starker Belastung oder gar einer Erkrankung, nicht ausreicht, ist die Unterstützung der endogenen Regulationsmechanismen zur Wiederherstellung der Barrierefunktion der Haut durch topisch applizierbare Externa unerläßlich.

Durch Umwelteinflüsse, insbesondere durch Lösungsmittel in vielen Nagellackentfernern, aber auch durch häufigen Kontakt zu anderen flüssigen Reingungsmitteln, z.B. Spül-, Wasch- und Putzmittel, können die Gesundheit der Nägel und deren äußeres Erscheinungsbild erheblich beeinträchtigt werden.

Nagelpflegeprodukte sollen Finger- und Zehennägel reinigen und ihnen eine ansprechende Erscheinung geben. Im speziellen Falle ist Ziel der Nagelpflege die Wiederherstellung und Förderung eines normalen Funktionsbildes der Fuß- und Fingernägel.

Insbesondere werden zur Nagelpflege Nagellacke verwendet, aber auch spezielle Nagelpflegeformulierungen, haufig auf der Basis kosmetischer oder dermatologischer Emulsionen, werden verwandt.

Die meisten Nagelpflegeprodukte des Standes der Technik haben sich jedoch als unzureichend erwiesen, da die hornigen Schichten der Nägel nur schwer von den meisten Nagelpflegeprodukten penetriert werden können und meist keine Inhaltsstoffe enthalten, die den hauteigenen Lipidmetabolismus ausreichend unterstützen.

Es hat sich überraschenderweise gezeigt und darin liegt die Lösung der Aufgabe, daß kosmetische und/oder dermatologische Wirkstoffkombinationen, bestehend aus
a) einer wirksamen Menge an Biotin und/oder Biotinestern,
b) einer wirksamen Menge an einer oder mehreren anderen α-Hydroxycarbonsäuren der allgemeinen Formel 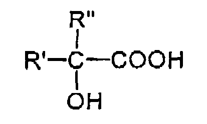 wobei R' und R" unabhängig voneinander gewählt werden aus der Gruppe
   (b1) H-,
   (b2) verzweigtes oder unverzweigtes C₁₋₂₅-Alkyl-,
   (b3) mit einer oder mehreren Carboxylgruppen und/oder Hydroxygruppen und/oder A-Idehydgruppen und/oder Oxogruppen (Ketogruppen) substituiertes verzweigtes oder unverzweigtes C₁₋₂₅-Alkyl-
   (b4) Phenyl-,
   (b5) mit einer oder mehreren Carboxylgruppen und/oder Hydroxygruppen und/oder verzweigten und/oder unverzweigten C₁₋₂₅-Alkylgruppen substituiertes Phenyl-,
      oder wobei das α-Kohlenstoffatom der α-Hydroxycarbonsäure mit R' und R" zusammen eine
   (b6) unsubstituierte Cycloalkylgruppe mit 3 bis 7 Ringatomen oder eine
   (b7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
   ausbildet, und
   wobei die α-Hydroxycarbonsäure oder die α-Hydroxycarbonsäuren gegebenenfalls in Form ihrer physiologisch verträglichen Salze und/oder Ethylester und/oder Methylester vorliegen können
   und
c) einer wirksamen Menge an einem oder mehreren Antioxidantien, welche gewählt werden aus der Gruppe bestehend aus den verschiedenen Ubichinonen und α-Liponsäure den Mißständen des Standes der Technik abhelfen.

Zwar ist aus der Schritt "Ärztliche Kosmetologie" Bd. 16, Nr.1 (1986), S. 47-54 (G.Faulhaber u. W.Lechner: "Der Einfluß von sebarned® flüssig Waschemulsion auf die ekzernatöse Haut") eine Rezeptur mit einem Gehalt an Biotin und Citronensäure bekannt, welche sich jedoch nicht durch einen Gehalt an Antioxidantien auszeichnet, und welche den Weg zur vorliegenden Erfindung nicht zu weisen instande war.

Biotin (D-cis-Hexahydro-2-oxothieno[3,4-d]imidazol-4-valeriansäure) wird gelegentlich auch Vitamin H (Hautvitamin) genannt, obwohl es heute zu den Vitaminen der B-Gruppe gerechnet wird (Vitamin B₇). Es ist allerdings im engeren Sinne kein Vitamin, da es von der menschlichen Darmflora synthetisiert werden kann. Es ist jedoch als prosthetische Gruppe (als solche auch "Coenzym R" genannt) der Enzyme, die Carboxylierungen und Decarboxylierungen im Organismus katalysieren (Biotin-Enzyme) unentbehrlich. Therapeutisch wird

Biotin beispielsweise zur Behandlung seborrhoischer Dermatitis bei Kleinkindern eingesetzt.

Biotin und seine Ester sind durch folgende generische chemische Strukturformel gekennzeichnet :

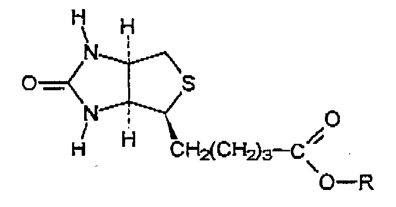

Erfindungsgemäß bedeutet R = H, verzweigtes oder unverzweigtes C₁₋₁₈-alkyl- oder verzweigtes oder unverzweigtes C₁₋₁₈-Alkenyl-, Bevorzugt sind solche Strukturen, bei welchen R = H, Methyl-, Ethyl darstellt.

Die erfindungsgemäßen α-Hydroxycarbonsäuren werden vorteilhaft gewählt aus folgenden Substanzklassen:
(b2) α-Hydroxyfettsäuren, wobei diese wiederum besonders vorteilhaft aus der Gruppe der C₁₀₋₁₈ -Alkylcarbonsäuren gewählt werden.
(b3) α-Hydroxyzuckersäuren, aliphatische α-Hydroxytruchtsäuren,
(b4) unsubstituierte aromatische α-Hydroxycarbonsäuren (z.B. Mandelsäure) bzw.
(b5) substituierte aromatische α-Hydroxycarbonsäuren.

Die unter Punkt (b2) fallenden α-Hydroxyfettsäuren werden besonders vorteilhaft gewählt aus der Gruppe
- α-Hydroxycarbonsäuren, gemäß der Formel 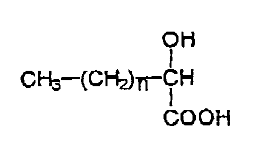 und/oder
- α-Hydroxy-isocarbonsäuren, gemäß der Formel 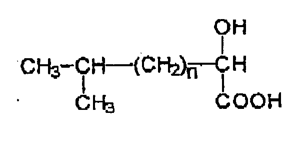 und/oder
- α-Hydroxy-anteisocarbonsäuren, gemäß der Formel 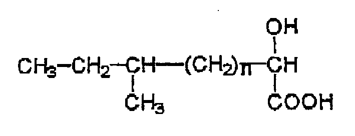
wobei n jeweils eine Zahl von 7 bis 31 darstellt.

Besonders vorteilhaft im Sinne der vorliegenden Erfindung ist, α-Hydroxycarbonsäuren zu verwenden, welche C₁₅-Körper darstellen, die also am α-Kohlenstoffatom eine verzweigtes oder unverzweigte C₁₄H₂₉-Kette tragen.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (b3) fallenden α-Hydroxyzuckersäuren werden besonders vorteilhaft gewählt aus der Gruppe der
- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (b3) fallenden aliphatischen α-Hydroxyfruchtsäuren werden besonders vorteilhaft gewählt aus der Gruppe Äpfelsäure. Milchsäure, Citronensäure, Weinsäure.

Apfelsäure (Hydroxybernsteinäure) ist durch folgende chemische Struktur gekennzeichnet

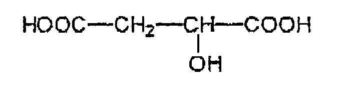

Milchsäure (2-Hydroxypropansäure) ist durch folgende chemische Struktur gekennzeichnet

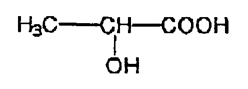

Citronensäure (2-Hydroxy-1,2,3-propantricarbonsäure) ist durch folgende chemische Struktur gekennzeichnet

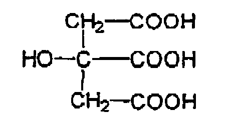

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet

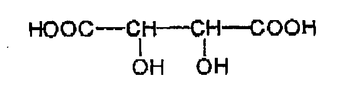

Insbesondere solche Wirkstoffkombinationen sind vorteilhaft, bei denen das Gewichtsverhältnis von Biotin und oder dessen

Estern zu der oder den α-Hydroxycarbonsäuren, insbesondere der oder den α-Hydroxyfruchtsäuren, bevorzugt der Citronensäure, im Bereich von 1 : 500 bis 20 : 1 liegt.

Besonders vorteilhafte Ausführungsformen der vorliegenden Erfindung betreffen kosmetische und/oder dermatologische Zubereitungen mit
a) einer wirksamen Menge an Biotin und/oder Biotinestern,
b) einer wirksamen Menge an Citronensäure und
c) einer wirksamen Menge an einem Ubichinon oder α-Liponsäure

Bevorzugt sind solche Zubereitungen mit einem Gehalt von

| | |
|---|---|
| 0,001 - 0,20 Gew.-% | an Biotin und/oder Biotinestern, |
| 0,001 - 5,00 Gew.-% | an einer oder mehreren α-Hydroxycarbonsäuren und/oder α-Ketocarbonsäuren und |
| 0,001 - 10,00 Gew.-% | an einem Ubichinon oder α-Liponsäure |

jeweils bezogen auf das Gesamtgewicht der Zubereitungen.

Besonders bevorzugt sind solche Zubereitungen mit einem Gehalt von

| | |
|---|---|
| 0,001 - 0,20 Gew.-% | an Biotin und/oder Biotinestern, |
| 0,001 - 5,00 Gew.-% | an Citronensäure und |
| 0,001 - 10,00 Gew.-% | an einem Ubichinon oder α-Liponsäure |

jeweils bezogen auf das Gesamtgewicht der Zubereitungen.

Es ist vorteilhaft, die Konzentration Ubichinon oder α-Liponsäure von aus dem Bereich von 0,001 - 2,00 Gew.-%, bezogen auf das Gesamtgewicht der Formulierung, zu wählen.

Erfindungsgemäß ist auch die Verwendung kosmetischer und/oder dermatologischer Wirkstoffkombinationen
a) einer wirksamen Menge an Biotin und/oder Biotinestern,
b) einer wirksamen Menge an einer oder mehreren α-Hydroxycarbonsäuren und/oder α-Ketocarbonsäuren und
c) einer wirksamen Menge an einem Ubichinon oder α-Liponsäure
zur Herstellung kosmetischer und/oder dermatologischer Zubereitungen zur Pflege der Haut und/oder der Hautanhangsgebilde.

Erfindungsgemäß ist insbesondere die Verwendung kosmetischer und/oder dermatologischer Wirkstoffkombinationen
a) einer wirksamen Menge an Biotin und/oder Biotinestern.
b) einer wirksamen Menge an Citronensäure und
c) einer wirksamen Menge an einem Ubichinon oder α-Liponsäure
zur Herstellung kosmetische und/oder dermatologischer Zubereitungen zur Pflege der Haut und/oder der Hautanhangsgebilde.

Erstaunlich war darüberhinaus, daß die erfindungsgemäßen Wirkstoffkombinationen bzw. kosmetischen und/oder dermatologischen Zubereitungen imstande sind, das Erscheinungsbild der Altershaut, welches vor allem durch Mangel an hauteigenen Lipiden gekennzeichnet ist, zu verbessern.

Es wird angenommen, daß die erfindungsgemäßen Wirkstoffkombinationen die körpereigenen Fähigkeiten stimulieren, Hautfette oder deren biochemische Vorläufer zu synthetisieren und den Lipidmetabolismus im positiven Sinne zu regulieren.

Erfindungsgemäß ist daher auch die Verwendung kosmetischer und/oder dermatologischer Wirkstoffkombinationen
a) einer wirksamen Menge an Biotin und/oder Biotinestern,
b) einer wirksamen Menge an einer oder mehreren α-Hydroxycarbonsäuren und/oder α-Ketocarbonsäuren und
c) einer wirksamen Menge an Ubichinon oder α-Liponsäure
zur Herstellung kosmetischer und/oder dermatologischer Zubereitungen zur Verbesserung des Erscheinungsbildes der trockenen Haut.

Erfindungsgemäß ist insbesondere die Verwendung kosmetischer und/oder dermatologischer Wirkstoffkombinationen
a) einer wirksamen Menge an Biotin und/oder Biotinestern,
b) einer wirksamen Menge an Citronensäure und
c) einer wirksamen Menge an Ubichinon oder α-Liponsäure
zur Herstellung kosmetischer und/oder dermatologischer Zubereitungen zur Verbesserung des Erscheinungsbildes der trockenen Haut.

Insbesondere sind die erfindungsgemäßen Wirkstoffkombinationen sowie kosmetische und/oder dermatologische Zubereitungen, solche Wirkstoffkombinationen enthaltend, imstande, das Erscheinungsbild der trockenen Haut zu mildern und Patienten, die unter diesem Erscheinungsbild leiden, zu einem günstigeren Hautfettstatus verhelfen. Erfindungsgemäß ist daher auch die Verwendung kosmetischer und/oder dermatologischer Wirkstoffkombinationen
a) einer wirksamen Menge an Biotin und/oder Biotinestern,
b) einer wirksamen Menge an einer oder mehreren α-Hydroxycarbonsäuren
c) einer wirksamen Menge an Ubichinon oder α-Liponsäure
bzw. die Verwendung kosmetischer und/oder dermatologischer Wirkstoffkombinationen
a) einer wirksamen Menge an Biotin und/oder Biotinestern,
b) einer wirksamen Menge an Citronensäure und
c) einer wirksamen Menge an Ubichinon oder α-Liponsäure
zur Herstellung kosmetischer und/oder dermatologischer Zubereitungen zur Verbesserung des Erscheinungsbildes der Altershaut.

Erfindungsgemäß ist ferner die Verwendung kosmetischer und/oder dermatologischer Wirkstoffkombinationen
a) einer wirksamen Menge an Biotin und/oder Biotinestern,
b) einer wirksamen Menge an einer oder mehrerer α-Hydroxycarbonsäuren und
c) einer wirksamen Menge an Ubichinon oder α-Liponsäure bzw.

a) einer wirksamen Menge an Biotin und/oder Biotinestern,
b) einer wirksamen Menge an Citronensäure und Ubichinon oder α-Liponsäure
c) einer wirksamen Menge an Ubichinon oder α-Liponsäure
zur Herstellung kosmetischer und/oder dermatologischer Zubereitungen zur Stimulierung der körpereigenen Fähigkeiten, Hautfette oder deren biochemische Vorläufer zu synthetisieren.

Schließlich ist es eine vorteilhafte Verkörperung der vorliegende Erfindung, die erfindungsgemäßen Zusammensetzungen, bzw. Zubereitungen diese enthaltend, zur Pflege der Haut bei dem Erscheinungsbild der atopischen Dermatits und/oder des seborrhoischen Ekzems anzuwenden.

Es ist dem Fachmanne natürlich bekannt, daß anspruchsvolle kosmetischen Zusammensetzungen zumeist nicht ohne die üblichen Hilfs- und Zusatzstoffe senkbar sind. Darunter zählen beispielsweise Konsistenzgeber, Füllstoffe, Parfum. Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhart, die erfindungsgemäßen Zusammensetzungen als Grundlage für pharmazeutische formulierungen zu verwenden.

Erfindungsgemäß ist auch die Verwendung der Wirkstoffkombinationen in hautpflegenden kosmetischen und/oder dermatologischen Zubereitungen.

Zur Anwendung werden die erfindungsgemäßen kosmetischen und dermatologischen Zubereitungen in der für Kosmetika üblichen Weise auf die Haut und/oder die Hautanhangsgebilde in ausreichender Menge aufgebracht

Besonders günstig sind solche kosmetischen und dermatologischen Zubereitungen, die in der Form eines Sonnenschutzmittels vorliegen. Vorzugsweise enthalten diese neben den erfindungsgemäßen Wirkstoffkombinationen zusäzlich mindestens einen UVA-Filter und/oder mindestens einen UVB-Filter und/oder mindestens ein anorganisches Pigment.

Erfindungsgemäße kosmetische und dermatologische Zubereitungen zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emission vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflachenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen
- öse, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäße Emulsionen, z.B. in Form einer Hautschutzcrème, einer Hautlotion, einer kosmetischen Milch, beispielsweise in Form einer Sonnenschutzcreme oder einer Sonnenschutzmilch, sind vorteilhart und enthalten z.B. die genannten Fette, Öle, Wachse und anderen Fettkörper, sowie Wasser und einen Emulgator, wie er üblicherweise für einen solchen Typ der Formulierung verwendet wird.

Erfindungsgemäße Gele enthalten üblicherweise Alkohole niedriger C-Zahl, z.B. Ethanol, isopropanol, 1,2-propandiol,

Glycerin und Wasser bzw, ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Erfindungsgemäße feste Stifte enthalten z.B. natürliche oder synthetische Wachse, Fettalkohole oder Fettsaureester. Bevorzugt werden Lippenpflegestifte.

Als Treibmittel für erfindungsgemäße, aus Aerosolbehältern versprühbare kosmetische und/oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet waren, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und/oder dermatologischen Zubereitungen zum Schutze der Haut enthalten

| | |
|---|---|
| 0,001 - 0,20 Gew.-% | an Biotin und/oder Biotinestern, |
| 0,001 - 5,00 Gew.-% | an einer oder mehreren α-Hydroxycarbonsäuren, insbesondere Citronensäure, und |
| 0,001 -10,00 Gew.-% | an Ubichinon oder α-Liponsäure |

jeweils bezogen auf das Gesamtgewicht der Zubereitungen.

Bevorzugt können sie außerdem Substanzen enthalten, die UVStrahlung im UVB-Bereich absorbieren, wobei die Gesamtmenge der Filtersubstanzen z.B. 0.1 Gew.-% bis 30 Ges.-% vorzugsweise 0.5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Sallcylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfönsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Kombination eines erfindungsgemäßen UVA-Filters mit einem UVB-Filter bzw. eine erfindungsgemaßes kosmetische oder dermatologische Zubereitung, welche auch einen UVB-Filter enthält.

Es kann auch von Vorteil sein, die erfindungsgemäßen Wirkstoffkombinationen mit UVA-Filtern zu kombinieren, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert-Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen, enthaltend die erfindunssgemäßen Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Bei erfindungsgemäßen kosmetischen und dermatologischen Zubereitungen für die Verwendung am Haar handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen und/oder dermatologischen Zubereitungen enthalten gegebenenfalls zusätzliche Wirkstoffe, Hilfs- und/oder Zusatzstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens. Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte sowie zusätzliche Substanzen zum Rückfetten der Haare bzw. der Kopfhaut.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, eine wirksame Menge an erfindungsgemäßen Wirkstoffkombinationen, und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschniten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder waßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Erfindungsgemäße kosmetische Zubereitungen zur Behandlung und Pflege der Haare, die erfindungsgemäße Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organische Komponenten. Diese Emulsionen enthalten gegebenenfalls kationische oberflächenaktive Substanzen.

Kosmetische Zubereitungen zur Behandlung und Pflege der Haare können als Gele vorliegen, die neben einem wirksamen Gehalt an den erfindungsgemäßen Wirkstoffkombinationen und dafür üblicherweise verwendeten Lösungsmitteln, bevorzugt Wasser, noch organische Verdickungsmittel, z.B. Gummiarabikum, Xanthangummi, Natriumalginat. Cellulose-Derivate, Vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdikkungsmittel ist in dem Gel z.B. in einer Menge zwischen 0.1 und 30 Cew.-%, bevorzugt zwischen 0.5 und 15 Gew.-%, enthalten.

Vorzugsweise wird die Menge der erfindungsgemäßen Wirkstoffkombinationen so gewählt, daß die entsprechenden Zubereitungen zur Behandlung und Pflege der Haare

| | |
|---|---|
| 0,001 - 0.20 Gew.-% | an Biotin und/oder Biotinestern. |
| 0.001 - 5,00 Gew.-% | an einer oder mehreren α-Hydroxycarbonsäuren und/oder α-Ketocarbonsäu ren, insbesondere Citronensäure, und |
| 0,001 - 10,00 Gew.-% | an Ubichinon oder α-Liponsäure |

jeweils bezogen auf das Gesamtgewicht der Zubereitungen, enthalten

Erfindungsgemäße kosmetische und dermatologische Zubereitungen zur Pflege und Wiederherstellung der Fuß- und Fingernagel können in verschiedenen Formen vorliegen, wie sie z.B. üblicherweise in diesem Typ von Zubereitungen eingesetzt werden.

So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Besonders bevorzugt liegen diese Zubereitungen in Form von Emulsionen vor.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe. Pigmente, die eine färbende Wirkung haben, Verdickungsmittel, oberflächenaktive Substanzen, Emulgatoren, weichmachende, anfeuchtende und/oder feuchhaltende Substanzen. Fette, Öle, Wachse oder andere übliche Bestandteile einer kosmetischen oder dermatologischen Formulierung wie Alkohole. Polyole, Polymere, Schaumstabilisatoren, Elektrolyte, organische Lösungsmittel oder Silikonderivate.

Sofern die kosmetische und/oder dermatologische Zubereitung zur Pflege oder Wiederherstellung der Nägel eine Lösung oder Lotion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester Von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Losungsmittel verwendet. Bei alkoholischen Lösungsmitteln kann Wasser ein weiterer Bestandteil sein.

Erfindungsgemäße Emulsionen zur Nagelpflege enthalten z.B. die genannten Fette, Öle, Wachse und anderen Fettkörper, sowie Wasser und einen Emulgator, wie er üblicherweise für einen solchen Typ der Formulierung verwendet wird.

Erfindungsgemäße Gele zur Pflege und/oder Wiederherstellung der Nägel enthalten üblicherweise Alkohole niedriger C-Zahl,
z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Es ist aber auch möglich und gegebenenfalls vorteilhart, die erfindungsgemäßen Wirkstoffkombinationen in Nagellacken, Nagellackentfernern, Nagelhärtern oder Nagelhautentfernern einzusetzen.

Erfindungsgemäße Nagellacke können Filmbildner, Harze, Lasungsmittel, sowie Hitfs- und Zusatzstoffe enthalten,

Nagelhärter können Substanzen enthalten, welche die freien Aminogruppen des Nagelkeratins vernetzt.

Nagelhautentferner können Wirkstoffe enthalten, welche die Nagelhaut erweichen, um deren Entfernung zu erleichtern.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Vorzugsweise wird die Menge der erfindungsgemäßen Wirkstoffkombinationen so gewählt daß die entsprechenden kosmetischen und/oder dermatologischen Zubereitungen zur Behandlung und Pflege der Finger- und Fußnägel

| | |
|---|---|
| 0,001 - 0,20 Gew.-% | an Biotin und/oder Biotinestern, |
| 0,001 - 5,00 Gew.-% | an einer oder mehreren α-Hydroxycarbonsäuren, insbesondere Citronensäure, und |
| 0,001 -10,00 Gew.-% | an Ubichinon oder α-Liponsäure |

jeweils bezogen auf das Gesamtgewicht der Zubereitungen, enthalten.

Gegenstand der Erfindung ist auch das Verfahren zur Herstellung der erfindungsgemäßen kosmetischen und/oder dermatologischen Zubereitungen, das dadurch gekennzeichnet ist, daß man in an sich bekannter Weise Biotin und/oder Biotinester, vorzugsweise in einer Menge entsprechend 0,001 - 0,20 Gew.-% an einer oder mehreren α-Hydroxycarbonsäuren, insbesondere Citronensäure, vorzugsweise in einer Menge entsprechend 0,001 - 5,00 Gew.-%, und Ubichinon oder α-Liponsäure, vorzugsweise in einer Menge entsprechend 0.001 - 5,00 Gew.-% jeweils bezogen auf das Gesamtgewicht der Zubereitungen, in kosmetische und/oder dermatologische Formulierungen einarbeitet.

Das nachfolgende Beispiel soll die vorliegende Erfindung verdeutlichen, ohne sie einzuschranken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel

| Handschutzcrème | | Gew.-% |
|---|---|---|
| A: | Polyglycerin-2-sesquiisostearat + Bienenwachs + Mineralöl + Magnesiumstearat ("Hostacerin W/O", Hoechst AG) | 10,00 |
| | Ceresin ("Lunacera M", Fuller) | 1,00 |
| | Dimethicone ("Silikonöl AK 100", Wacker) | 2,00 |
| | α-Liponsäure | 0,50 |
| | Vaseline DAB 9 | 5,00 |
| | Paraffinöl DAB 9 | 7,00 |
| B: | Parfum | q.s. |
| | Biotin ("D-Biotin", Hoffmann-Laroche) | 0,05 |
| C: | Glycerin | 3,00 |
| | Citronensäure | 0,50 |
| | Konservierungsmittel | q.s. |
| | Wasser, VES | ad 100,00 |

Die Bestandteile der Phasen A bzw. C werden jewels zusammengemischt die beiden Phasen bei 75° C vereinigt und emulgiert. Das emulgierte Gemische aus A und C wird auf 40° C abgekühft und mit der ebenfalls auf 40° C erwärmten Phase B vereinigt und kaltgerührt.

## Patentansprüche

1. Kosmetische und/oder dermatologische Wirkstoffkombinationen, bestehend aus
a) einer wirksamen Menge an Biotin und/oder Biotinestern,
b) einer wirksamen Menge an einer oder mehreren anderen α-Hydroxycarbonsäuren der allgemeinen Formel 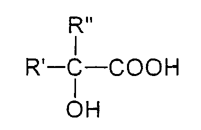 wobei R' und R" unabhängig voneinander gewählt werden aus der Gruppe
(b1) H-,
(b2) verzweigtes oder unverzweigtes C₁₋₂₅-Alkyl-,
(b3) mit einer oder mehreren Carboxylgruppen und/oder Hydroxygruppen und/oder A-Idehydgruppen und/oder Oxogruppen (Ketogruppen) substituiertes verzweigtes oder unverzweigtes C₁₋₂₅-Alkyl-
(b4) Phenyl-,
(b5) mit einer oder mehreren Carboxylgruppen und/oder Hydroxygruppen und/oder verzweigten und/oder unverzweigten C₁₋₂₅-Alkylgruppen substituiertes Phenyl-,
oder wobei das α-Kohlenstoffatom der α-Hydroxycarbonsäure mit R' und R" zusammen eine
(b6) unsubstituierte Cycloalkylgruppe mit 3 bis 7 Ringatomen oder eine
(b7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
ausbildet, und
wobei die α-Hydroxycarbonsäure oder die α-Hydroxycarbonsäuren gegebenenfalls in Form ihrer physiologisch verträglichen Salze und/oder Ethylester und/oder Methylester vorliegen können
und
c) einer wirksamen Menge an einem oder mehreren Antioxidantien, welche gewählt werden aus der Gruppe bestehend aus den verschiedenen Ubichinonen und α-Liponsäure.

2. Wirkstoffkombinationen nach Anspruch 1, wobei das Gewichtsverhältnis von Biotin und/oder dessen Estern zu der oder den α-Hydroxycarbonsäuren im Bereich von 1 : 500 bis 20 : 1 liegt.

3. Wirkstoffkombinationen nach Anspruch 1, **dadurch gekennzeichnet, daß** die Biotinester gewählt werden aus der Gruppe, die durch folgende Strukturen **gekennzeichnet** ist: 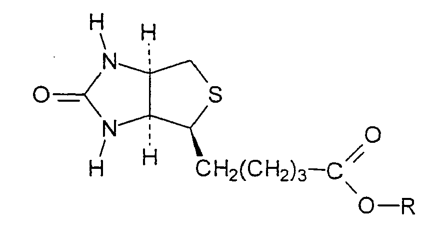 wobei R: H, verzweigtes oder unverzweigtes C₁₋₁₈-Alkyl- oder verzweigtes oder unverzweigtes C₁₋₁₈-Alkenyl- bedeutet.

4. Wirkstoffkombinationen nach Anspruch 1, **dadurch gekennzeichnet, daß** die α-Hydroxysäuren gewählt werden aus der Gruppe
(b2) α-Hydroxyfettsäuren
(b3) α-Hydroxyzuckersäuren, aliphatische α-Hydroxyfruchtsäuren,
(b4) unsubstituierte aromatische α-Hydroxycarbonsäuren
(b5) substituierte aromatische α-Hydroxycarbonsäuren.

5. Wirkstoffkombinationen nach Anspruch 1, **dadurch gekennzeichnet, daß** die α-Hydroxysäuren gewählt werden aus der Gruppe
(b2)
- α-Hydroxycarbonsäuren, gemäß der Formel 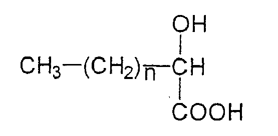
- α-Hydroxy-isocarbonsäuren, gemäß der Formel 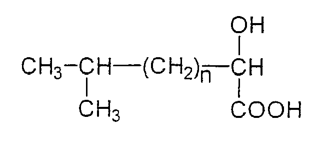 und/oder
- α-Hydroxy-anteisocarbonsäuren, gemäß der Formel 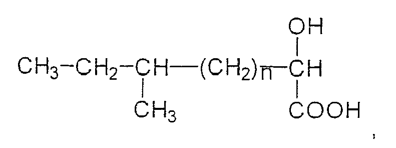
wobei n jeweils eine Zahl von 7 bis 31 darstellt,
(b3)
- Aldonsäuren, Aldarsäuren, Uronsäuren, Glycerinsäure, Äpfelsäure, Milchsäure, Essigsäure, Citronensäure.

6. Kosmetische und/oder dermatologische Zubereitungen, enthaltend eine wirksame Menge einer Wirkstoffkombination gemäß Anspruch 1.

7. Zubereitungen nach Anspruch 6, **dadurch gekennzeichnet, daß** sie einen Gehalt von
| | |
|---|---|
| 0,001 - 0,20 Gew.-% | an Biotin und/oder Biotinestern, |
| 0,001 - 5,00 Gew.-% | an einer oder mehreren α-Hydroxycarbonsäuren |
| 0,001 - 10,00 Gew.-% | an einem Antioxidans oder mehreren Antioxidantien, gewählt aus der in Anspruch 1 genannten Gruppe |
aufweisen.

8. Verwendung von Wirkstoffkombinationen gemäß Anspruch 1 zur Herstellung kosmetischer und/oder dermatologischer Zubereitungen zur Pflege der Haut und/oder der Hautanhangsgebilde.

9. Verwendung von Wirkstoffkombinationen gemäß Anspruch 1 zur Herstellung kosmetischer und/oder dermatologischer Zubereitungen zur Verbesserung des Erscheinungsbildes der trockenen Haut.

10. Verwendung von Wirkstoffkombinationen gemäß Anspruch 1 zur Herstellung kosmetischer und/oder dermatologischer Zubereitungen zur Verbesserung des Erscheinungsbildes der Altershaut.

11. Verwendung von Wirkstoffkombinationen gemäß Anspruch 1 zur Herstellung kosmetischer und/oder dermatologischer Zubereitungen zur Stimulierung der körpereigenen Fähigkeiten, Hautfette oder deren biochemische Vorläufer zu synthetisieren.

12. Verwendung von Wirkstoffkombinationen gemäß Anspruch 1 zur Herstellung kosmetischer und/oder dermatologischer Zubereitungen zur Pflege der Haut beim Erscheinungsbild der atopischen Dermatitis und/oder des seborrhoischen Ekzems.

## Claims

1. Cosmetic and/or dermatological active compound combinations comprising
a) an active amount of biotin and/or biotin esters,
b) an active amount of one or more other α-hydroxycarboxylic acids of the general formula 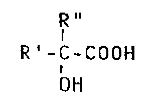 wherein R' and R" independently of one another are chosen from the group consisting of
(b1) H-,
(b2) branched or unbranched C₁₋₂₅-alkyl-,
(b3) branched or unbranched C₁₋₂₅-alkyl- which is substituted by one or more carboxyl groups and/or hydroxyl groups and/or aldehyde groups and/or oxo groups (keto groups),
(b4) phenyl- and
(b5) phenyl- which is substituted by one or more carboxyl groups and/or hydroxyl groups and/or branched and/or unbranched C₁₋₂₅-alkyl groups,
or wherein the α-carbon atom of the α-hydroxycarboxylic acid, together with R' and R", forms a
(b6) unsubstituted cycloalkyl group having 3 to 7 ring atoms or a
(b7) cycloalkyl group having 3 to 7 ring atoms which is substituted by one or more carboxyl groups and/or hydroxyl groups and/or oxo groups (keto groups) and/or branched and/or unbranched C₁₋₂₅-alkyl groups,
and
wherein the α-hydroxycarboxylic acid or the α-hydroxycarboxylic acids can optionally be present in the form of their physiologically tolerated salts and/or ethyl esters and/or methyl esters,
and
c) an active amount of one or more antioxidants which are chosen from the group consisting of the various ubiquinones and α-lipoic acid.

2. Active compound combinations according to Claim 1, wherein the weight ratio of biotin and/or esters thereof to the α-hydroxycarboxylic acid or acids is in the range from 1 : 500 to 20 : 1.

3. Active compound combinations according to Claim 1, **characterized in that** the biotin esters are chosen from the group **characterized by** the following structures: 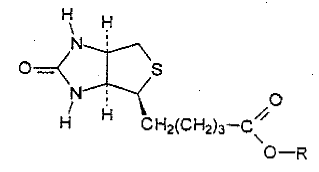 wherein R is H, branched or unbranched C₁₋₁₈-alkyl- or branched or unbranched C₁₋₁₈-alkenyl-,

4. Active compound combinations according to Claim 1, **characterized in that** the α-hydroxy acids are chosen from the group consisting of
(b2) α-hydroxy-fatty acids,
(b3) α-hydroxy-sugar acids or aliphatic α-hydroxy-fruit acids,
(b4) unsubstituted aromatic α-hydroxycarboxylic acids and
(b5) substituted aromatic α-hydroxycarboxylic acids.

5. Active compound combinations according to Claim 1, **characterized in that** the α-hydroxy acids are chosen from the group consisting of
(b2)
- α-hydroxycarboxylic acids according to the formula 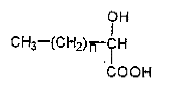
- α-hydroxy-isocarboxylic acids according to the formula 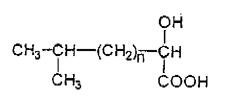 and/or
- α-hydroxy-anteisocarboxylic acids according to the formula 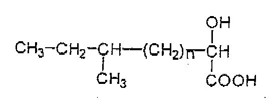
wherein n in each case is a number from 7 to 31, and
(b3)
- aldonic acids, aldaric acids, uronic acids, glyceric acid, malic acid, lactic acid, acetic acid and citric acid.

6. Cosmetic and/or dermatological formulations comprising an active amount of an active compound combination according to Claim 1.

7. Formulations according to Claim 6, **characterized in that** they contain
| | |
|---|---|
| 0.001 - 0.20% | by weight of biotin and/or biotin esters, |
| 0.001 - 5.00% | by weight of one or more α-hydroxycarboxylic acids |
| 0.001 - 10.00% | by weight of an antioxidant or several antioxidants chosen from the group mentioned in Claim 1. |

8. Use of active compound combinations according to Claim 1 for the preparation of cosmetic and/or dermatological formulations for care of the skin and/or the integumentary appendages.

9. Use of active compound combinations according to Claim 1 for the preparation of cosmetic and/or dermatological formulations for improving the appearance of dry skin.

10. Use of active compound combinations according to Claim 1 for the preparation of cosmetic and/or dermatological formulations for improving the appearance of aged skin.

11. Use of active compound combinations according to Claim 1 for the preparation of cosmetic and/or dermatological formulations for stimulating the endogenous capacities for synthesis of skin fats or biochemical precursors thereof.

12. Use of active compound combinations according to Claim 1 for the preparation of cosmetic and/or dermatological formulations for care of the skin with the manifestation of atopic dermatitis and/or seborrhoeic eczema.

## Revendications

1. Combinaisons de principes actifs cosmétiques et/ou dermatologiques, constituées de
a) une quantité active de biotine et/ou d'esters de biotine,
b) une quantité active d'un ou de plusieurs autres acides α-hydroxycarboxyliques de formule générale 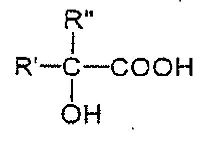 dans laquelle R' et R " , indépendamment l'un de l'autre, sont choisis parmi le groupe constitué de
(b1) un atome d'hydrogène,
(b2) un groupe alkyle en C₁₋₂₅ ramifié ou non ramifié,
(b3) un groupe alkyle en C₁₋₂₅ ramifié ou non ramifié, substitué par un ou plusieurs groupes carboxyle et/ou groupes hydroxy et/ou groupes aldéhyde et/ou groupes oxo (groupes céto),
(b4) un groupe phényle,
(b5) un groupe phényle substitué par un ou plusieurs groupes carboxyle et/ou groupes hydroxy et/ou groupes alkyle en C₁₋₂₅ ramifiés et/ou non ramifiés,
ou l'atome de carbone en α de l'acide α-hydroxycarboxylique formant, conjointement avec R' et R" ,
(b6) un groupe cycloalkyle non substitué renfermant de 3 à 7 atomes du noyau ou
(b7) un groupe cycloalkyle renfermant de 3 à 7 atomes du noyau, substitué par un ou plusieurs groupes carboxyle et/ou groupes hydroxy et/ou groupes oxo (groupes céto) et/ou groupes alkyle en C₁₋₂₅ ramifiés et/ou non ramifiés,
et
l'acide α-hydroxycarboxylique ou les acides α-hydroxycarboxyliques pouvant éventuellement se présenter sous la forme de leurs sels physiologiquement acceptables et/ou de leurs esters éthyliques et/ou de leurs esters méthyliques,
et
c) une quantité active d'un ou de plusieurs agents anti-oxydants qui sont choisis parmi le groupe constitué des diverses ubiquinones et de l'acide α-lipoique,

2. Combinaisons de principes actifs selon la revendication 1, dans lesquelles le rapport pondéral entre la biotine et/ou ses esters et le ou les acides α-hydroxycarboxyliques est dans la plage de 1 : 500 à 20 : 1.

3. Combinaisons de principes actifs selon la revendication 1, **caractérisées en ce que** les esters de biotine sont choisis parmi le groupe qui est **caractérisé par** les structures suivantes : 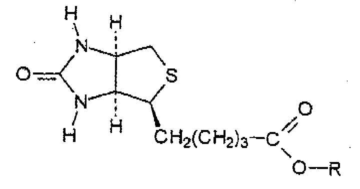 dans lesquelles R représente un atome d'hydrogène, un groupe alkyle en C₁₋₁₈ ramifié ou non ramifié ou un groupe alcényle en C₁₋₁₈ ramifié ou non ramifié.

4. Combinaisons de principes actifs selon la revendication 1, **caractérisées en ce que** les acides α-hydroxycarboxyliques sont choisis parmi le groupe constitué de
(b2) des acides gras α-hydroxy,
(b3) des acides de sucre α-hydraxy, des acides de fruit α-hydroxy aliphatiques,
(b4) des acides α-hydroxycarboxyligues aromatiques non substitués,
(b5) des acides α-hydroxycarboxyliques aromatiques substitués.

5. Combinaisons de principes actifs selon la revendication 1, **caractérisées en ce que** les acides α-hydroxycarboxyliques sont choisis parmi le groupe constitué de
(b2)
- des acides α-hydroxycarboxyliques selon la formule 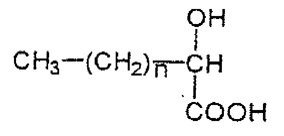
- des acides α-hydroxy-isocarboxyliques selon la formule 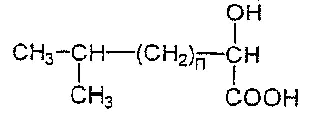 et/ou
- des acides α-hydroxy-anté-isocarboxyliques selon la formule 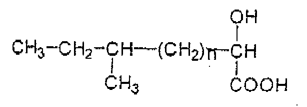
dans lesquelles n représente, dans chaque cas, un nombre de 7 à 31,
(b3)
- des acides aldoniques, des acides aldariques, des acides uroniques, l'acide glycérique, l'acide malique, l'acide
lactique, l'acide acétique et l'acide citrique.

6. Formulations cosmétiques et/ou dermatologiques comprenant une quantité active d'une combinaison de principes actifs selon la revendication 1.

7. Formulations selon la revendication 6, **caractérisées en ce qu'**elles contiennent
de 0,001 à 0,20 % en poids de biotine et/ou d'esters de biotine,
de 0,001 à 5,00 % en poids d'un ou de plusieurs acides α-hydroxycarboxyliques,
de 0,001 à 10,00 % en poids d'un agent antioxydant ou de plusieurs agents anti-oxydants choisis parmi le groupe mentionné dans la revendication 1.

8. Utilisation de combinaisons de principes actifs selon la revendication 1 pour la préparation de formulations cosmétiques et/ou dermatologiques pour les soins de la peau et/ou des éléments tégumentaires.

9. Utilisation de combinaisons de principes actifs selon la revendication 1 pour la préparation de formulations cosmétiques et/ou dermatologiques pour améliorer l'aspect de la peau sèche.

10. Utilisation de combinaisons de principes actifs selon la revendication 1 pour la préparation de formulations cosmétiques et/ou dermatologiques pour améliorer l'aspect de la peau vieillie.

11. Utilisation de combinaisons de principes actifs selon la revendication 1 pour la préparation de formulations cosmétiques et/ou dermatologiques pour stimuler les aptitudes endogènes à la synthèse de corps gras de la peau ou de leurs précurseurs biochimiques.

12. Utilisation de combinaisons de principes actifs selon la revendication 1 pour la préparation de formulations cosmétiques et/ou dermatologiques pour les soins de la peau dans le cas d'une manifestation de la dermatite atopique et/ou de l'eczéma séborrhéique.
